# EUROPEAN PATENT APPLICATION

(11) **EP 3 366 215 A1**
(43) Date of publication of application: **29.08.2018**
(21) Application number: 16857772.4
(22) Date of filing: 19.10.2016
(51) Int. Cl.: A61B 6/00, A61B 6/10

(54) **X-RAY DETECTOR AND SYSTEM FOR SENSING BENDING OF SAME**

(30) Priority: 19.10.2015 KR 20150145277; 19.10.2015 KR 20150145278
(71) Applicant: Rayence Co., Ltd., Hwaseong-si, Gyeonggi-do 18449 (KR); Vatech Ewoo Holdings Co., Ltd., Hwaseong-si, Gyeonggi-do 18449 (KR)
(72) Inventor: JEON, Yu Sung, Hwaseong-si Gyeonggi-do 18449 (KR)
(74) Representative: Markfort, Iris-Anne Lucie
(86) International application number: PCT/KR2016/011758
(87) International publication number: WO 2017/069514

(57) **Abstract**

The present invention provides an X-ray detector having bendable characteristics and including pressure sensing means for measuring the magnitude of an applied external pressure.

## Description

### Technical Field

The present invention relates to an X-ray detector. More particularly, the present invention relates to an X-ray detector being bendable and allowing easy detection of damage thereto caused by an external force, to an X-ray detector being flexible and allowing easy measurement of the degree of bending thereof, and to a bending detection system for detecting bending of the X-ray detectors.

### Background Art

In the medical field, such as dentistry, digital X-ray detectors are widely used in place of traditional photographic film X-ray detectors.

Conventional digital X-ray detectors are non-compliant or unbendable because they are made of a rigid material, which may cause discomfort for certain patients during intraoral radiography.

To solve this problem, X-ray detectors that are flexible have been intensively studied and developed. However, conventional flexible X-ray detectors are easily damaged by excessive bending caused by an external force during handling or intraoral radiography, or by being bitten by a patent during intraoral radiography.

However, at present, it is impossible to check flexible X-ray detectors for damage. Therefore, there is no way to determine whether the damage of a flexible X-ray detector is caused by a manufacturer during the manufacturing process or by an external force in a place of use such as a clinic or hospital. Therefore, it is difficult to clarify who is responsible for the damage to the flexible X-ray detector.

In addition, excessive bending may cause damage to a flexible X-ray detector during handling or use. Therefore, a measure for checking for the degree of bending of a flexible X-ray detector is required to prevent the excessive bending which may result in damage to the flexible X-ray detector. However, at present, there is no method of measuring the degree of bending of the flexible X-ray detector during use.

### Disclosure

### Technical Problem

An objective of the present invention is to provide a method of easily checking for damage of a flexible X-ray detector attributable to an external force.

Another objective of the present invention is to provide a method of easily measuring the degree of bending of a flexible X-ray detector.

### Technical Solution

In order to accomplish the above objectives, according to one aspect of the present invention, there is provided an X-ray detector configured to be flexible, the X-ray detector equipped with a pressure sensor configured to measure the magnitude of an external force applied thereto.

The pressure sensor may be configured to change in color according to the magnitude of the external force.

The X-ray detector may further include a sensor panel configured to convert an incident X-ray into an electrical signal, and the pressure sensor may be disposed at a front side or a rear side of the sensor panel.

The pressure sensor may be directly attached to the sensor panel.

The X-ray detector may further include: a first casing configured to cover a front portion of the sensor panel and to limit an elastic bending of the sensor panel; a second casing disposed in front of the first casing and configured to accommodate the sensor panel and the first casing; and a bending control member disposed at the rear side of the sensor panel and configured to control the elastic bending of the sensor panel.

The flexible X-ray detector may further include a soft housing member covering at least part of an outer surface of the X-ray detector.

According to another aspect of the present invention, there is provided a bending detection system for detecting bending of an X-ray detector, the system including: an X-ray detector configured to be flexible; a bending sensor provided in the X-ray detector and configured to vary in resistance according to the degree of bending of the X-ray detector; a signal generator circuit generating a bending signal corresponding to a change in the resistance; a bending information generator circuit generating and transmitting a bending information signal in accordance with the bending signal to an output device.

The signal generator circuit includes a resistor connected in series with the bending sensor to configure a voltage divider circuit in conjunction with the bending sensor and an analog-digital converter circuit converting an output voltage of the voltage divider circuit to a digital signal serving as the bending signal.

The signal generator circuit may further include an operational amplifier that amplifies the output voltage of the voltage divider circuit and outputs the resulting amplified signal to the analog-digital converter circuit.

The bending information generator circuit generates a voice information signal indicating the degree of bending and transmits the resulting voice information signal to a voice output device. Alternatively, the bending information generator circuit may generate a display information signal indicating the degree of bending and transmits the display information signal to a display device.

The X-ray detector may include a first casing configured to cover a front portion of the sensor panel and to limit the elastic bending of the sensor panel.

The X-ray detector may further include a bending control member disposed at a rear side the sensor panel and controlling the elastic bending of the sensor panel.

### Advantageous Effects

According to the present invention, the pressure sensor sheet that can measure the magnitude of a pressure applied thereto is provided in the X-ray detector configured to be flexible.

Accordingly, it is possible to accurately and easily measure the magnitude of an external force applied to the X-ray detector. Therefore, it is possible to effectively determine whether the X-ray detector is damaged by an external force or not.

Therefore, it is possible to determine whether the damage of the X-ray detector is caused by a manufacturer during manufacturing or by an external pressure during use thereof in a place such as a hospital. Therefore, it is possible to clarify who is responsible for the damage of the X-ray detector.

According to the present invention, since the bending sensor is provided in the X-ray detector, it is possible to easily and accurately measure the degree of bending of the X-ray detector and to inform a user of the degree of bending of the X-ray detector. Therefore, the user can easily perceive the degree of bending of the X-ray detector, thereby being able to prevent the X-ray detector from being permanently damaged by excessive bending.

### Description of Drawings

FIG. 1 is a perspective view illustrating an X-ray detector according to a first embodiment of the present invention;
FIG. 2 is an exploded perspective view of the X-ray detector of FIG. 1;
FIG. 3 is a perspective view illustrating the schematic construction of a sensor assembly according to the first embodiment of the present invention;
FIG. 4 is a cross-sectional view illustrating the schematic construction of an indirect conversion sensor panel according to the first embodiment of the present invention;
FIG. 5 is a schematic view illustrating changes in concentration of a color according to changes in external force applied to a pressure sensor sheet according to the first embodiment of the present invention;
FIG. 6 is a perspective view schematically illustrating a first casing according to the first embodiment of the present invention;
FIG. 7 is a flowchart illustrating a sequence of determining the cause of damage of the X-ray detector by using the pressure sensor sheet according to the first embodiment of the present invention;
FIG. 8 is a schematic diagram illustrating a bending detection system for detecting bending of an X-ray detector according to a second embodiment of the present invention;
FIG. 9 is a diagram illustrating an equivalent circuit of a bending sensor and a signal generator circuit according to the second embodiment of the present invention;
FIG. 10 is a perspective view illustrating an X-ray detector according to the second embodiment of the present invention;
FIG. 11 is an exploded perspective view of the X-ray detector of FIG. 10;
FIG. 12 is a perspective view illustrating the schematic construction of a sensor assembly according to the second embodiment of the present invention;
FIG. 13 is a cross-sectional view illustrating the schematic construction of an indirect conversion sensor panel according to the second embodiment of the present invention;
FIG. 14 is a perspective view schematically illustrating a first casing according to the second embodiment of the present invention; and
FIG. 15 is a diagram illustrating a state in which the X-ray detector according to the second embodiment of the present invention is bent.

### Mode for Invention

Hereinafter, embodiments of the present invention will be described in detail with reference to the accompanying drawings.

### <First Embodiment>

An X-ray detector according to a first embodiment of the present invention is a flexible X-ray detector being flexible and bendable. Hereinafter, for convenience of a description, as an example of an X-ray detector configured to be flexible, a flexible X-ray detector used as an intraoral sensor will be described.

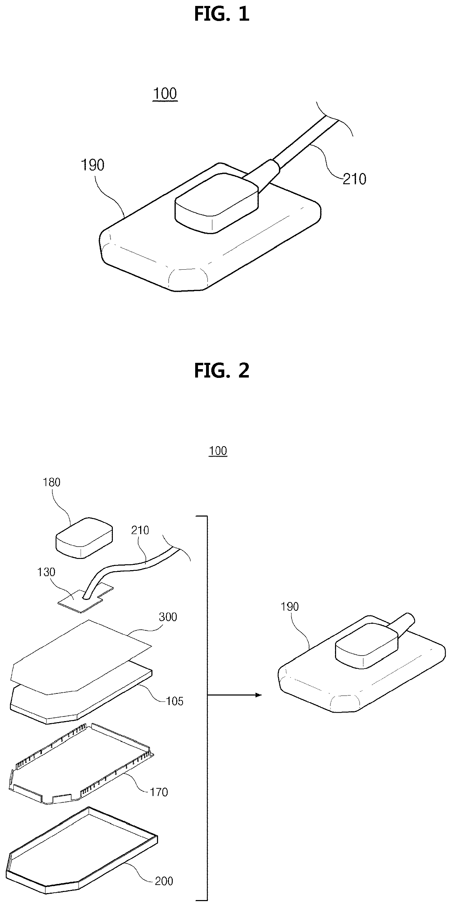

FIG. 1 is a perspective view of the X-ray detector according to the first embodiment of the present invention and FIG. 2 is an exploded perspective view of the X-ray detector of FIG. 1.

Referring to FIGS. 1 and 2, according to the first embodiment of the present invention, an X-ray detector 100 includes a sensor assembly 105 including a sensor panel that detects an incident X-ray and generates an electrical signal corresponding to the detected X-ray, a printed circuit board (PCB) 130 disposed at a rear side of the sensor assembly 105 and connected to the sensor assembly 105, a first casing 170 disposed at a front side (i.e., an X-ray incidence) of the sensor assembly 105, a back-surface support 180 disposed at the rear side (opposite to the X-ray incidence side) of the sensor assembly 105, a second casing 200 in which the sensor assembly 105 and the first casing 170 are received, and a housing member 190 that encloses the X-ray detector 100.

In addition, the X-ray detector 100 according to the first embodiment further includes a pressure sensor sheet 300 attached to either the front surface or the rear surface of the sensor assembly 105 as a pressure sensing unit.

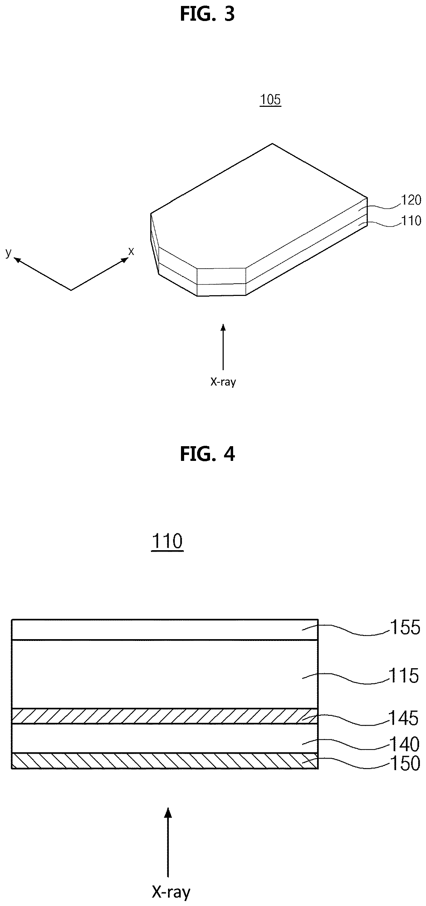

The sensor assembly 105 will be described in greater detail below with reference to FIG. 3 as well as FIGS. 1 and 2. FIG. 3 is a perspective view illustrating the schematic construction of the sensor assembly 105 according to the first embodiment of the present invention.

Referring to FIG. 3 as well as FIGS. 1 and 2, the sensor assembly 105 is configured with a sensor panel 110. Alternatively, the sensor assembly 105 may further include a bending control member 120, as necessary. The sensor panel 100 and the bending control member 120 are preferably arranged in a direction in which an X-ray travels but the arrangement thereof is not limited thereto.

The sensor panel 110 includes an effective region (also, referred to as an active region) for acquisition of an X-ray image. In the active region, multiple pixels are arranged in columns and rows, i.e., in a matrix form. Each pixel includes a photoelectric transducer such as a photodiode and a switching device and converts incident light into an electrical signal for transmission. Although not illustrated in detail, pads through which the electrical signals are output are disposed at a periphery portion of the sensor panel 110. The switching device may be configured with a complementary metal-oxide semiconductor (CMOS) transistor or a field emission transistor (TFT).

In order for the X-ray detector 100 to be flexible, the sensor panel 110 also needs to be flexible. To this end, the sensor panel 110 is preferably formed to have a thickness of 100 µm or less on the premise that it is made of a brittle substrate, such as a semiconductor substrate, a ceramic substrate, or a glass substrate. Particularly, when the sensor panel 110 is made of a semiconductor substrate, it preferably has a thickness of 30 to 90 pm. With such a thickness range, the sensor panel 110 may have an optimum bending strength.

To fabricate the sensor panel 110 having a predetermined thickness within the above range, a back surface portion of a prepared raw substrate may be eliminated so that the substrate may be thinned to a predetermined thickness. For example, as a method of thinning the substrate, mechanical grinding, chemical polishing, or plasma etching may be performed on the back surface of the substrate, in which the back surface is a surface where the photoelectric transducers are not formed.

As the sensor panel 110, a direct conversion sensor panel that directly converts an incident X-ray into an electrical signal, or an indirect conversion sensor panel that first converts an incident X-ray into visible light and subsequently converts the visible light into an electrical signal may be used.

In the case where an indirect conversion sensor panel is used as the sensor panel, as illustrated in FIG. 4 that schematically illustrates the sensor panel 110 of the present embodiment, a scintillator layer 140 may be formed on one surface of a substrate 115 of the sensor panel 110, i.e., on the photoelectric transducers, to convert an incident X-ray into visible light, in comparison with the case where a direct conversion sensor panel is used.

In the example of FIG. 4, the scintillator layer 140 is provided on an X-ray incidence surface of the sensor panel 110. However, alternatively, the scintillator layer 140 may be provided on the opposite surface.

The scintillator layer 140 may be attached to the substrate 115 using an adhesive layer 145. In addition, a protective film 150 made of an X-ray radiation transmissible material is provided on the scintillator layer 140 to protect the scintillator layer 140. The adhesive layer 145 may be made of a flexible adhesive having a high light transmittance. For example, it may be made of an optically clear adhesive (OCA) film. The protective film 150 may be made of a resin film that is highly radiation-transmissible and highly moisture-proof. The adhesive layer 145 may have a thickness of 5 to 50 µm so as to compensate for the brittleness of the sensor panel. Preferably, the OCA film may have a thickness of 10 to 40 pm.

The scintillator layer 140 may be made of a phosphor based on CsI or gadolinium oxysulphide (called Gadox, Gd₂O₂:Tb).

Since the X-ray detector 100 according to the present embodiment needs to be flexible, Gadox may be advantageously used over CsI having a columnar crystalline grain structure. Since the Gadox-based phosphor has a fine particle structure, when the X-ray detector 100 is bent, the possibility of breakage is very low, so that the X-ray detector 100 may have no or few defects even though it is bent by an external force. Another advantage of the Gadox-based scintillator layer 140 is that manufacture thereof is easier.

The Gadox-based scintillator layer 140 is formed to have a thickness of 50 to 300 µm to be sufficiently X-ray radiation transmissible. Preferably, its thickness may range from 70 to 200 pm. In this case, an additional protective film made of a radiation-transmissible and moisture-proof resin may be provided between the scintillator layer 140 and the adhesive layer 145 for the purposes of protection and supporting of the scintillator layer 140. A total thickness of the laminate of the scintillator layer 140, the protective film 150, and the additional protective film may be within a range of 250 to 500 µm and, preferably, in a range of 300 to 450 pm. However, the total thickness is not limited thereto.

In addition, a flexible layer 155 may be formed on the back surface of the substrate 115 with the scintillator layer 140 formed on the front surface thereof. The flexible layer 155 may be made of a resin having a flexible property, for example, polyimide (PI). The flexible layer 155 has sufficient thickness to compensate for the brittleness of the sensor panel 110 and specifically for the brittleness of the substrate 115 to prevent the X-ray detector 100 from being broken when the X-ray detector 100 is bent by an external force. For example, the flexible layer 155 has a thickness of 50 to 150 pm. The flexible layer 155 may be attached to the substrate 115 using a predetermined bonding material such as a die attach film (IDAF). Preferably, the bonding material is 10 to 30 µm thick.

The bending control member 120 has substantially the same shape as the sensor panel 110 to cover the entire area of the back surface of the sensor panel 110. The bending control member 120 is preferably made of an elastic material having elasticity higher than that of the sensor panel 110. Therefore, the bending control member 120 limits the bending of the sensor panel 110 to below the maximum bending strength thereof. That is, the sensor panel 110 can be bent to the extent that the maximum bending strength of the bending control member 120 allows. The sensor assembly 105 is bendable and reversible within a range limited by the elasticity of the bending control member 120. The bending control member 120 functions to absorb a portion of the stress of the sensor panel 110, attributable to the bending thereof, thereby reducing the brittleness of the sensor panel 110 when the sensor assembly 105 is bent.

To this end, the bending control member 120 may be made of a composite resin material containing two or more substances. Preferably, the bending control member 120 may be made of a composite resin containing a reinforcing agent and a resin.

The bending control member 120 is preferably formed such that a bending characteristic for a first horizontal direction and a second horizontal direction that are on the same plane and perpendicular to each other, differ.

Regarding this point, when the sensor assembly 105 has a planar rectangular shape in which an X-axis length is longer than a Y-axis length, the bending control member 120 is preferably formed such that its X-axis bending strength is larger than its Y-axis bending strength. On the other hand, when the sensor assembly 105 has a substantially square shape, the bending control member may be configured such that its bending strengths for different directions (an X-axis direction and a Y-axis direction) perpendicular to each other differ.

With such a bending characteristic, the sensor assembly 105 has ability to bend more flexibly in a longer-axis direction than a shorter-axis direction, which effectively reduces the discomfort which may be caused in the mouth of a patient during intraoral radiography.

Regarding the discomfort which may occur during intraoral radiography, edges of the X-ray detector 100 may be the main cause of the discomfort and, specifically, the longer-axis direction distal end of the X-ray detector 100 is most problematic. For this reason, the sensor assembly 105 features an increased bending strength thereof and, specifically, features that the longer-axis bending strength is larger, which greatly reduces patient discomfort.

Since the bending control member 120 has a larger x-axis (longer-axis) direction bending strength than a Y-axis (shorter-axis) direction bending strength, its torsion stress is mostly transferred in the X-axis direction, which is helpful to prevent the sensor panel 110 (specifically, the substrate 115) from being broken.

The bending control member 120 having different bending strengths for different directions on the same plane may be made of a composite resin, for example, a fiber reinforced polymer (FRP) containing a fiber reinforcing agent. FRP is a material obtained by adding a reinforcing agent to a resin base. Examples of the reinforcing agent include inorganic fibers, such as glass fiber, carbon fiber, and boron fiber, and organic fibers, such as aramid fiber, polyester fiber, and Kevlar® fiber. Examples of the resin base include thermoplastic resins, such as such as unsaturated polyester, epoxy, phenol, and polyimide, and thermosetting resins, such as polyamide, polycarbonate, ABS, PBT, PP, or SAN.

The sensor assembly 105 includes a printed circuit board (PCB) 130 disposed at a rear portion thereof. The printed circuit board 130 is electrically connected to the sensor panel 110 via a flexible printed circuit film (for example, FPC), thereby being able to receive an electrical signal from the sensor panel 110 and to transmit a drive signal to the sensor panel 110.

The printed circuit board 130 may be connected to a signal transmission cable 210 so as to exchange an electrical signal with an external device.

As the printed circuit board 130, a flexible printed circuit board made of a flexible material may be used to impart a flexible property to the sensor assembly 110.

Since the printed circuit board 130 is part of the sensor assembly 105, it may have a size occupying a portion of the sensor assembly 105. For example, the printed circuit board 130 has a size corresponding to a center portion of the sensor assembly 105. However, the size of the printed circuit board 130 is not limited thereto. The printed circuit board 130 may have a size as large as the entire area of the sensor assembly 105. In this case, the printed circuit board 130 may be attached to the bending control member 120.

According to the present embodiment, a pressure sensor sheet 300 may be provided either on the front surface or on the back surface of the sensor assembly 105. To avoid being interfered with by X-ray radiation, the pressure sensor sheet 300 is preferably provided on the back surface of the sensor assembly 105. In the present embodiment, an example in which the pressure sensor sheet 300 is provided on the back surface of the sensor assembly 105 will be described.

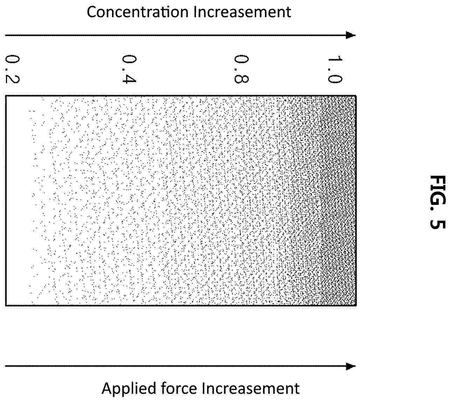

The pressure sensor sheet 300 is an element to detect the magnitude of an external force applied to the sensor panel 110 of the X-ray detector 100, and changes in color according to the magnitude of the applied force. For example, the concentration of a color may change according to the magnitude of the applied force. Regarding this point, as illustrated in FIG. 5, the concentration of color becomes thicker as the magnitude of the applied force increases.

Therefore, when an external force is applied to the X-ray detector 100, thereby causing the X-ray detector 100 to be bent during handling or during intraoral radiography, or when the X-ray detector 100 is bitten by a patient' teeth during intraoral radiography, the color of a portion of the pressure sensor sheet 300, to which the external force is applied, changes. Therefore, it is possible to determine the position at which the external force is applied within the pressure sensor sheet 300, and the magnitude of the external force applied to the position, based on the change in color of the pressure sensor sheet 300.

In terms of obtaining a better approximation to the force exerted on the sensor panel 110, the pressure sensor sheet 300 is preferably disposed as close as possible to the surface of the sensor panel 110. For example, in the case where the bending control member 105 is included, the pressure sensor sheet is interposed between the bending control member 105 and the sensor panel 110 and is preferably directly attached to the sensor panel 110.

The first casing 170 provided at the front side of the sensor assembly 105 has a box shape with a rear side being opened, and serves as a window cover that allows X-rays to pass therethrough. The sensor assembly 105 may be accommodated in the first casing 170.

The first casing 170 is attached to the sensor assembly 105 using a radiation transmissible adhesive. For the radiation transmissible adhesive, an OCA or a foam tape, may be used, but the adhesive is not limited thereto.

The first casing 170 constructed as described above protects the sensor assembly 105 and, particularly, a front side of the sensor panel 110.

Particularly, according to the present embodiment, the first casing 170 functions to define and limit an overall bending strength of the X-ray detector 100 by its structural design.

In regard to this point, the first casing 170 is made of a material having a high strength and being highly flexible. For example, the first casing 170 is made of a resin, a flexible glass, or an FRP, but the material of the first casing 170 is not limited thereto. The first casing 170 may have a thickness of 0.1 to 0.5 mm, but the thickness of the first casing 170 may not be limited thereto.

Since the first casing 170 is formed in a manner described above, the degree of bending of the sensor assembly 105 may be limited to below the maximum bending strength of the first casing 170. Therefore, it is possible to prevent the sensor assembly 105 from being excessively bent, thereby preventing the sensor panel 110 which is a key element of the sensor assembly 105 from being broken. In addition, since the X-ray detector 100 is bent only to the extent that is under a predetermined limit, the distortion of an obtained X-ray image is reduced.

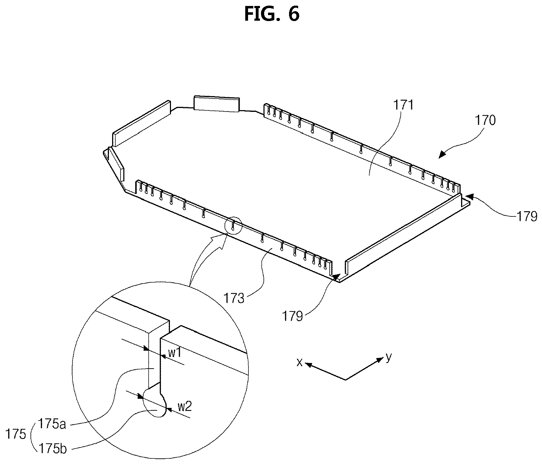

The structure of the first casing 170 will be described in more detail with reference to FIG. 6. The first casing 170 includes a base portion 171 and side walls 173 extending rearward perpendicularly from the respective edges of the back surface of the base portion 171.

The base portion 171 may have a substantially flat plate shape. Preferably, the side walls 173 are not provided at corners of the base portion 171. That is, the side walls 173 adjacent to each other are not connected to each other but are spaced from each other with a gap 179 therebetween, at the corresponding corner of the base portion.

As described above, the side walls 173 are not continuous along the outer periphery of the base portion but are separated from each other with the gaps 179 at the respective corners. Therefore, it is possible to reduce the structural resistance induced at the corners of the first casing 170 and to prevent the first casing 170 from being broken by stress which is usually likely to concentrate on the corners.

The side walls 173 are provided with a plurality of slit-like recesses arranged in a lengthwise direction thereof.

Specifically, among the four side walls 173, two opposite side walls 173 extending along the X-axis (longer-axis) direction of the first casing 170 and facing each other are provided with the recesses 175. The recess formed in one side wall (hereinafter, referred to as a first side wall to differentiate it from the other side wall among the two recesses-provided sidewalls) and the recess formed in the other side wall (hereinafter, referred to as a second side wall) are formed to correspond to each other. The recesses 175 are arranged such that the interval between adjacent recesses is relatively large at a middle portion in the longitudinal direction of the side wall and decreases with a distance to each end.

With the recesses 175 arranged in a manner described above, the degree of bending changes according to positions in the X-axis direction. The smaller the interval of the recesses 175 at a position, the larger the degree of bending at the position. That is, the larger the interval of the recesses 175 at a position, the smaller the degree of bending at the position.

That is, the degree of bending increases as the distance to each end of the first casing 170 in the X-axis direction decreases. Therefore, the X-ray detector 100 has a bending characteristic in which the degree of bending varies according to positions in the X-axis direction.

By varying the bending degree according to positions, it is possible to effectively reduce patient discomfort during intraoral radiography.

Actually, respective ends of the X-ray detector 100 frequently come into contact with the intraoral tissues rather than the center portion, so that the respective ends of the X-ray detector 100 mainly cause the discomfort. To solve this problem, the X-ray detector 100 is configured such that its respective end portions are more flexible, which may reduce the discomfort for patients. On the other hand, since a center portion of the X-ray detector 10 is relatively less flexible, the overall distortion of an X-ray image attributable to the bending of the X-ray detector is reduced.

Although the example in which the recesses 175 are formed in the side walls 173 extending in the longer-axis direction has been described above, the recesses 175 may be provided in the side walls 173 extending in the shorter-axis direction as well as the side walls 173 extending in the longer-axis direction. The recesses 175 provided in the side walls 173 extending shorter-axis direction also may vary in interval therebetween.

The recesses 175 provided in the side walls 173 are formed to extend down from the top of each side wall 173. The recesses 175 extending down from the top of the side wall may be stepped recesses, each including a first recess portion 175a having a first width w1 that is a constant width and a second recess portion 125b extending down from a lower end of the first recess portion 175a. At least part of the second recess portion has a second width w2 larger than the first width w1.

The second recess portion 175b may be formed in various shapes. The first embodiment provides an example in which the second recess portion 175b is circular.

The structure in which the second recess portion 175b has a larger width than the first recess portion has the following advantages: preventing a lower portion of the recess 175 provided in the side wall 173 from being damaged during bending of the X-ray detector 100; and improving the bending characteristic of the X-ray detector 100 by allowing the recesses 175 to be expanded more broadly when the X-ray detector 100 is bent.

Alternatively, the recesses 175 starting from the top of the side wall 173 may extend to reach the base portion 171. This case also provides the same advantages. Additionally, the base portion 171 may be provided with a plurality of recesses extending perpendicularly to the lengthwise direction of the X-ray detector 100 in either an outside surface or an inside surface of the base portion 171, as necessary. The surface provided with the recesses may be a surface that comes into contact with the sensor panel 110 or may be surface that does not come into contact with the sensor panel 110. The intervals of the recesses formed in the surface of the base portion 171 are set in the same manner as the recesses formed in the side walls. That is, the interval decreases with a distance to each end of the base portion. In this case, the recesses formed in the base portion 171 may have a groove form or a slot form that does not pass through the base portion 171 in the thickness direction. When the recesses are formed in the inside surface of the base portion 171, the recesses have a depth less than the thickness of the base portion 171 and are formed to taper to the bottom of the recesses.

The back-surface support 180 is disposed at a rear portion of the sensor assembly 105 and functions as a grip post for supporting the X-ray detector 100 during intraoral radiography. The back-surface support 180 is held by operator's fingers or is connected to an instrument such as an extension cone paralleling (XCP) system. The back-surface support 180 may be made of a high-strength resin such as polycarbonate (PC) and acrylonitrile Butadiene styrene (ABS).

The back-surface support 180 may be installed to cover the printed circuit board 130, thereby protecting an electrical contact between the signal transmission cable 210 and the printed circuit board 130 to maintain stable electrical contact therebetween.

The back-surface support 180 may be disposed at a center portion of the sensor assembly 105, thereby supporting the rear side of the sensor assembly 105. Therefore, the center portion of the sensor assembly 105 is reduced in the degree of bending compared to the other portions, for example, respective end portions of the sensor assembly 105.

Therefore, the center portion of the X-ray detector 100 is less flexible and the periphery portions of the X-ray detector 100 are more flexible. This characteristic provides effects of reducing the discomfort which may be caused to a patient and minimizing the distortion of an X-ray radiograph. As such, the back-surface support 180 limits the bending degree of the X-ray detector 100 at the position at which it is disposed, and controls the degree of bending of the X-ray detector 100 according to its arrangement position.

The second casing 200 is structured such that the sensor assembly 105 and the first casing 170 are received therein. The second casing 200 covers the front side and the outer flank surfaces of the first casing 170. Besides, the second casing 200 also may cover at least part of the back surface of the sensor assembly 105, as necessary.

The second casing 200 is preferably made of a resin, but the material of the second casing 200 is not limited thereto. Specifically, taking into consideration a bending characteristic that the limit is imposed on the degree of bending of the X-ray detector, the second casing 200 is preferably made of a material having a shore hardness of about D10 to D20, but the material of the second casing 200 is not limited thereto.

When the second casing 200 is made of a material that has the ability to bend within a limited range, it is possible to set the limit of the overall bending of the X-ray detector 100 as intended.

The X-ray detector 100 with the second casing 200 combined may undergo a molding process so that at least part of the X-ray detector 100 may be covered by a soft housing member 190. The housing member 190 surrounds the external surface of the X-ray detector 100, thereby protecting the X-ray detector 100.

The housing member 190 may be formed to cover the entire surface of the sensor assembly 105. Alternatively, the housing member 190 also may cover the second casing 200 and/or the back-surface support 180, as necessary.

The housing member 190 may be made of a soft material such as silicone or urethane. Preferably, the soft material for the housing member 190 may have a shore hardness of about A30 to A50. However, the soft material for the housing member 190 is not limited thereto.

When damage is caused to the X-ray detector 100 that is flexible, it is possible to determine whether the damage is caused by an external force by using the pressure sensor sheet 300.

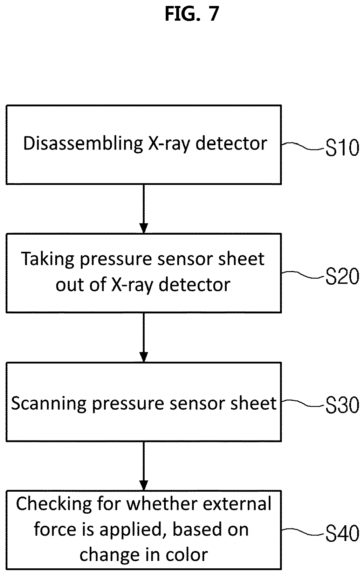

Regarding this point, a more detailed description will be given with reference to FIG. 7. First, the X-ray detector 100 is disassembled (S10). Next, the pressure sensor sheet 300 is taken out (S20).

Next, the pressure sensor sheet 300 is scanned using a scanner (S30). Next, the magnitude of the force applied to pressure sensor sheet 300 and the force-applied position of the pressure sensor sheet 300 are determined based on the change in color of the pressure sensor sheet 300 (S40).

Through the above procedure, it is possible to easily determine whether the damage of the X-ray detector 100 is caused by an external force.

As described above, according to the first embodiment of the present invention, the pressure sensor sheet having the ability to measure the magnitude of an applied force is provided in the X-ray detector.

Accordingly, it is possible to accurately and easily measure the magnitude of an external force applied to the X-ray detector. Therefore, it is possible to effectively determine whether the X-ray detector is damaged by an external force or not.

Therefore, it is possible to determine whether the damage of the X-ray detector is caused by a manufacturer during manufacturing or by an external force in a place of use, such as a hospital. Therefore, it is possible to clarify who is responsible for the damage to the X-ray detector.

### <Second Embodiment>

An X-ray detector according to a second embodiment of the present invention is a flexible X-ray detector. Hereinafter, for convenience of a description, as an example of a flexible X-ray sensor, a flexible X-ray detector used as an intraoral sensor will be described.

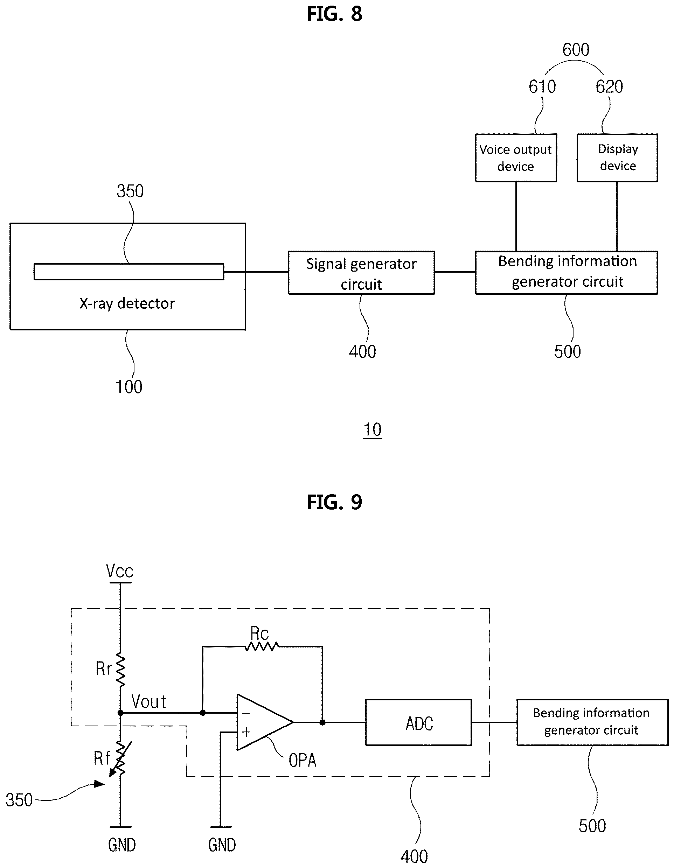

FIG. 8 is a schematic diagram illustrating a bending detection system for detecting bending of an X-ray detector according to the second embodiment of the present invention, and FIG. 9 is a diagram schematically illustrating an equivalent circuit of a bending sensor and a signal generator circuit according to the second embodiment of the present invention.

Referring to FIG. 8, according to the second embodiment of the present invention, a bending detection system 10 includes an X-ray detector 100, a bending sensor 350 mounted in the X-ray detector 100 and configured to be flexible, a signal generator circuit 400 generating a bending signal corresponding to the degree of bending of the bending sensor 350, and a bending information generator circuit 500 processing the bending signal and generating a bending information signal that is an output information signal to be output through an output device 600.

In FIG. 8, for convenience of a description, the signal generator circuit 400 and the bending information generator circuit 500 are provided outside the X-ray detector 100. However, at least one of the signal generator circuit 400 and the bending information generator circuit 500 may be mounted in the X-ray detector 100.

The X-ray detector 100 detects incident X-rays and outputs an electrical signal. The details of the X-ray detector 100 will be described below.

The bending sensor 350 is an element for measuring the degree of bending, i.e., a bending angle of the X-ray detector 100. As illustrated in FIG. 9, the bending sensor 350 is configured with a variable resistor (RF) that changes in resistance according to the bending angle thereof.

The bending sensor 350 is mounted in the X-ray detector 100, thereby being bent at the same time when the X-ray detector 100 is bent. Thus, the resistance value a variable resistor Rf of the bending sensor 350 changes in proportional to the bending angle of the X-ray detector 100. Thus, it is possible to measure the degree of bending (bending angle) of the X-ray detector 100, based on the change in the resistance value of the variable resistor Rf of the bending sensor 350.

The bending sensor 350 has a strip shape elongated in one direction, and the degree of bending of the X-ray detector 100 for the lengthwise direction in which the bending sensor 350 extends can be measured.

The signal generator circuit 400 connected to the bending sensor 350 includes a resistor (hereinafter, also referred to as a first resistor) Rr having a fixed resistance value and an analog-digital converter circuit (ADC). The signal generator circuit 400 may further include an operational amplifier (OPA) functioning as an output buffer.

The first resistor Rr is connected in series with the bending sensor 350 (i.e., the variable resistor Rf), thereby configuring a voltage divider circuit. Power supply voltages Vcc and GNC may be applied to the respective ends of the voltage divider circuit. In the present embodiment, a positive power supply voltage Vcc and a ground voltage GND are applied to the respective ends of the voltage divider circuit.

When the voltage divider circuit composed of the first resistor Rr and the variable resistor Rf is used, an output voltage (divided voltage) Vout is output from a node between the first resistor Rr and the variable resistor Rf. The output voltage Vout changes with the resistance value of the variable resistor Rf. That is, since the resistance of the variable resistor Rf changes with the degree of bending of the X-ray detector 100, the output voltage Vout of the voltage divider circuit changes with the degree of bending of the X-ray detector 100.

The output voltage Vout of the voltage divider circuit is an analog signal. Therefore, it may be converted into a digital signal by the analog-digital converter circuit ADC.

Alternatively, the output voltage Vout of the voltage divider circuit may be amplified by the operational amplifier OPA configured with a negative feedback circuit before being converted into the digital signal. In order to control the amplification factor of the operational amplifier OPA (i.e., the increased level of the output voltage Vout), a resistor (hereinafter, referred to as a second resistor) Rc may be connected between an output terminal and an inverted input terminal of the operational amplifier OPA.

The analog-digital converter circuit ADC outputs a digital signal corresponding to the input voltage. Consequently, the analog-digital converter circuit ADC outputs the digital signal serving as the bending signal corresponding to the degree of bending of the X-ray detector 100.

The bending signal generated by the signal generator circuit 400 is input to the bending information generator circuit 500. In response to this input signal, the bending information generator circuit 500 generates the bending information signal in accordance with the degree of bending.

The bending information signal is an output information signal to be transmitted to the output device 600 to inform the user of the degree of bending of the X-ray detector 100 via the output device 600. The bending information signal includes, for example, at least one of a voice information signal and a display information signal indicating the degree of bending. The present embodiment presents an example in which both of the voice information signal and the display information signal are generated.

In this case, the voice information signal indicating the degree of bending is transmitted to a voice output device 610 such as a speaker and the display information signal indicating the degree of bending is transmitted to a display device 620 such as a monitor device.

When voice information on the degree of bending of the X-ray detector 100 is output through the voice output device 610, the user can be aware of the degree of bending of the X-ray detector 100 in an audible manner. For example, the bending angle (the degree of the bending) of the X-ray detector 100 is excessively large, a warning voice message may be output.

When voice information on the degree of bending of the X-ray detector 100 is output through the voice output device 620, the user can be aware of the degree of bending of the X-ray detector 100 in an audible manner. For example, the bending angle (the degree of the bending) of the X-ray detector 100 is excessively large, a warning voice message may be output.

For reference, the bending information generator circuit 500 may create and record a log file containing the history of the change in the degree of bending of the X-ray detector 100 as well as outputting the bending information to the voice output device 610 and/or the display device 620.

As described above, according to the present embodiment, with the bending sensor 350 built in the X-ray detector 100, it is possible to easily and accurately detect the degree of bending and to provide the information on the degree of bending to user. Therefore, the user can monitor the degree of bending of the X-ray detector 100, thereby being able to prevent the X-ray detector 100 from being damaged by an excessive degree of bending.

Hereinafter, the X-ray detector according to the second embodiment of the present invention will be described in detail.

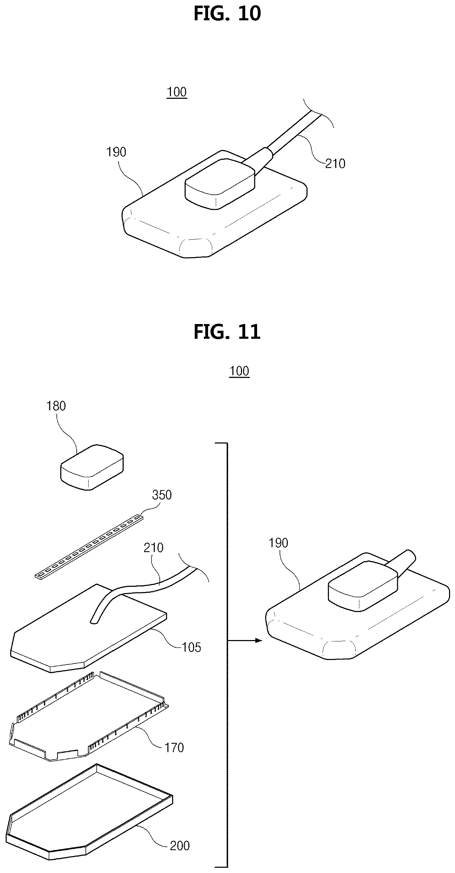

FIG. 10 is a perspective view of the X-ray detector according to the second embodiment of the present invention and FIG. 11 is an exploded perspective view of the X-ray detector of FIG. 10.

Referring to FIGS. 10 and 11, the X-ray detector 100 according to the present embodiment includes a sensor assembly 105 detecting X-rays and generating an electrical signal corresponding to the detected X-rays, and a bending sensor 350. The X-ray detector 100 further includes a first casing 170 disposed at a front side (X-ray incidence side) of the sensor assembly 105, a back-surface support 180 disposed at a rear side (opposite to the X-ray incidence side) of the sensor assembly 105, a second casing 200 in which the sensor assembly 105 and the first casing 170 are received, and a housing member 190 covering the X-ray detector 100.

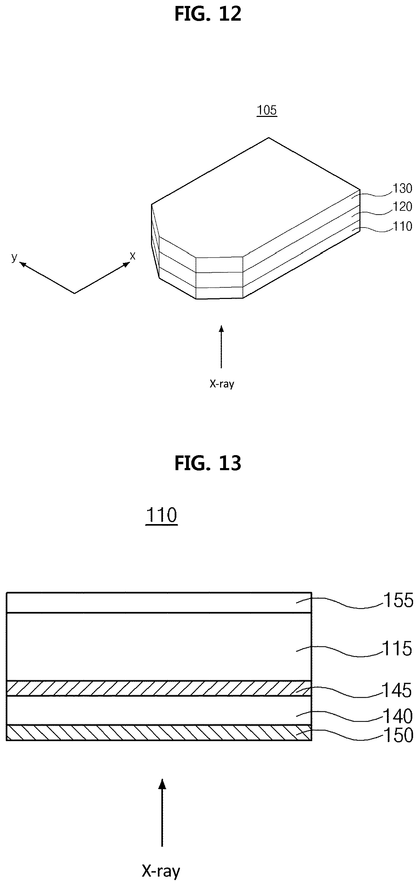

The sensor assembly 105 will be described in greater detail below with reference to FIG. 12 as well as FIGS. 10 and 11. FIG. 12 is a perspective view illustrating the schematic construction of the sensor assembly according to the second embodiment of the present invention.

Referring to FIGS. 12 as well as FIGS. 10 and 11, the sensor assembly 105 includes a sensor panel 110 and a printed circuit board 130. Alternatively, it may further include a bending control member 120, as necessary. The sensor panel 100, the bending control member 120, and the printed circuit board 130 are preferably arranged in a direction in which an X-ray travels but the arrangement thereof is not limited thereto.

The sensor panel 110 includes an effective region (also, referred to as an active region) for acquisition of an X-ray image. In the active region, multiple pixels are arranged in columns and rows, in a matrix form. Each pixel includes a photoelectric transducer such as a photodiode and a switching device and converts incident light into an electrical signal for transmission. Although not illustrated in detail, pads through which the electrical signals are output are disposed at a periphery portion of the sensor panel 110. The switching device may be configured with a complementary metal-oxide semiconductor (CMOS) transistor or a field emission transistor (TFT).

To allow the X-ray detector 100 to be flexible, the sensor panel 110 also needs to be flexible. To this end, the sensor panel 110 is preferably formed to have a thickness of 100 µm or less on the premise that it is made of a brittle substrate, such as a semiconductor substrate, a ceramic substrate, or a glass substrate. Particularly, when the sensor panel 110 is made of a semiconductor substrate, the sensor panel 110 preferably has a thickness of 30 to 90 pm. With such a thickness range, the sensor panel 110 may have an optimum bending strength.

To fabricate the sensor panel 110 having a predetermined thickness within the above range, a back surface portion of a prepared raw substrate may be removed so that the substrate may be thinned to a predetermined thickness. For example, as a method of thinning a substrate, mechanical grinding, chemical polishing, or plasma etching may be performed on the back surface of the substrate, in which the back surface is a surface opposite to a surface where the photoelectric transducers are formed.

Although the sensor panel 110 in the present embodiment is a direct conversion sensor panel that directly converts an incident X-ray into an electrical signal, an indirect conversion sensor panel that first converts an incident X-ray into visible light and subsequently converts the visible light into an electrical signal may be used as the sensor panel 110 in place of the direct conversion sensor panel.

In the case where an indirect conversion sensor panel is used as the sensor panel, as illustrated in FIG. 13 that is a cross-sectional view schematically illustrating the sensor panel 110 of the present embodiment, a scintillator layer 140 may be formed on one surface of a substrate 115 of the sensor panel 110, i.e., on the photoelectric transducers, to convert an incident X-ray into visible light, in comparison with the case where a direct conversion sensor panel is used.

In the example of FIG. 13, the scintillator layer 140 is provided on an X-ray incidence surface of the sensor panel 110. However, alternatively, the scintillator layer 140 may be provided on the opposite surface.

The scintillator layer 140 may be attached to the substrate 115 using an adhesive layer 145. In addition, a protective film 150 made of an X-ray radiation transmissible material is provided on the scintillator layer 140 to protect the scintillator layer 140. The adhesive layer 145 may be made of a flexible adhesive having a high optical transmittance. For example, it may be made of an optically clear adhesive (OCA) film. The protective film 150 may be made of a resin film that is highly radiation-transmissible and highly moisture-proof. The adhesive layer 145 may have a thickness of 5 to 50 µm so as to compensate for the brittleness of the sensor panel. Preferably, the OCA film may have a thickness of 10 to 40 pm.

As a phosphor constituting the scintillator layer 140, a phosphor based on CsI or Gadox (Gd₂O₂:Tb) may be used, for example.

Since the X-ray detector 100 according to the present embodiment needs to be flexible, Gadox may be advantageously used over CsI having a columnar crystalline grain structure. Since the Gadox-based phosphor has a fine particle structure, even though the X-ray detector 100 is bent, the possibility of breakage of the X-ray detector 100 is very low, resulting in the X-ray detector 100 having no or few defects attributable to the bending. Another advantage of the Gadox-based scintillator layer 140 is that manufacture thereof is easier.

The Gadox-based scintillator layer 140 is formed to have a thickness of 50 to 300 µm to be sufficiently X-ray radiation transmissible. Preferably, its thickness may range from 70 to 200 pm. In this case, an additional protective film made of a radiation-transmissible and moisture-proof resin may be provided between the scintillator layer 140 and the adhesive layer 145 to protect and support the scintillator layer 140. A total thickness of the laminate of the scintillator layer 140, the protective film 150, and the additional protective film may be within a range of 250 to 500 µm and, preferably, in a range of 300 to 450 pm. However, the total thickness is not limited thereto.

In addition, a flexible layer 155 may be formed on the back surface of the substrate 115 with the scintillator layer 140 formed on the front surface thereof. The flexible layer 155 may be made of a resin having a flexible property, for example, polyimide (PI). The flexible layer 155 has sufficient thickness to compensate for the brittleness of the sensor panel 110 and specifically for the brittleness of the substrate 115 to prevent the X-ray detector 100 from being broken when the X-ray detector 100 is bent by an external force. For example, the flexible layer 155 has a thickness of 50 to 150 pm. The flexible layer 155 may be attached to the substrate 115 using a predetermined adhesive material such as a die attach film (IDAF). Preferably, the adhesive material is formed to a thickness of 10 to 30 pm.

As the printed circuit board 130, a flexible printed circuit board made of a flexible material may be used to impart a flexible property to the sensor assembly 110.

The printed circuit board 130 is connected to the sensor panel 110 via a flexible printed circuit film (FPC), thereby communicating an electrical signal with the sensor panel 110. The printed circuit board 130 is connected to a signal transmission cable 210 to communicate an electrical signal with an eternal system. The printed circuit board 130 may have a thickness of 130 to 350 pm, allowing for the elasticity of the printed circuit board 130. However, the thickness of the printed circuit board 130 is not limited to this range. Any thickness of the printed circuit board 130, which provides an elasticity equal to or lower than that of the sensor panel 110, is allowable.

The bending control member 120 has substantially the same shape as the sensor panel 110 to cover the entire area of the back surface of the sensor panel 110. The bending control member 120 is preferably made of an elastic material having elasticity higher than that of the sensor panel 110. The bending control member 120 limits the bending of the sensor panel 110 to below the maximum bending strength thereof. That is, the sensor panel 110 can be bent to the extent that the maximum bending strength of the bending control member 120 allows. The sensor assembly 105 is bendable and reversible within a range limited by the elasticity of the bending control member 120. The bending control member 120 functions to absorb a portion of the stress of the sensor panel 110, attributable to the bending thereof, thereby reducing the brittleness of the sensor panel 110 when the sensor assembly 105 is bent.

To this end, the bending control member 120 may be made of a composite resin material containing two or more substances. Preferably, the bending control member 120 may be made of a composite resin containing a reinforcing agent and a resin.

The bending control member 120 is preferably formed such that a bending characteristic for a first horizontal direction and a second horizontal direction that are on the same plane and perpendicular to each other, differ.

Regarding this point, when the sensor assembly 105 has a planar rectangular shape in which an X-axis length is longer than a Y-axis length, the bending control member 120 is preferably formed such that its X-axis bending strength is larger than its Y-axis bending strength. On the other hand, when the sensor assembly 105 has a substantially square shape, the bending control member may be configured such that its bending strengths for different directions (an X-axis direction and a Y-axis direction) perpendicular to each other differ.

With this bending characteristics, the sensor assembly 105 has ability to bend more flexibly in a longer-axis direction than a shorter-axis direction, which effectively reduces patient discomfort during intraoral radiography.

Regarding the discomfort which may be caused during intraoral radiography, edges of the X-ray detector 100 may be the main cause of the discomfort and, specifically, the long-axis direction distal end of the X-ray detector 100 is a primary cause of the discomfort. For this reason, the sensor assembly 105 features an increased bending strength, specifically, features that its long-axis bending strength is larger, which greatly reduces a patient discomfort.

Since the bending control member 120 has a larger x-axis (longer-axis) direction bending strength than a Y-axis (shorter-axis) direction bending strength, its torsion stress is mostly transferred in the X-axis direction, which is helpful to prevent the sensor panel 110 (specifically, the substrate 115) from being broken.

The bending control member 120 having different bending strengths for different directions on the same plane may be made of a composite resin, for example, a fiber reinforced polymer (FRP) containing a fiber reinforcing agent. FRP is a material obtained by adding a reinforcing agent to a resin base. Examples of the reinforcing agent include inorganic fibers, such as glass fiber, carbon fiber, and boron fiber and organic fibers, such as aramid fiber, polyester fiber, and Kevlar® fiber. Examples of the resin base include thermoplastic resins, such as such as unsaturated polyester, epoxy, phenol, and polyimide, and thermosetting resins, such as polyamide, polycarbonate, ABS, PBT, PP, or SAN.

The first casing 170 provided at the front side of the sensor assembly 105 has a box shape with a rear face being opened, and serves as a window cover that allows X-rays to pass therethrough. The sensor assembly 105 is received in the first casing 170.

The first casing 170 and the sensor assembly 105 are attached using a radiation transmissible adhesive. As the radiation transmissible adhesive, an OCA or a foam tape may be used, but the usable adhesives are not limited thereto.

The first casing 170 constructed as described above protects the sensor assembly 105 and, particularly, the front side of the sensor panel 110.

Particularly, according to the present embodiment, the first casing 170 functions to define and limit an overall bending strength of the X-ray detector 100 by its structural design.

Regarding this point, the first casing 170 is made of a material having a high strength and a good bending characteristic. For example, the first casing 170 is made of a resin, a flexible glass, or an FRP, but the material of the first casing 170 is not limited thereto. The first casing 170 may have a thickness of 0.1 to 0.5 mm, but the thickness of the first casing 170 is not limited thereto.

Since the first casing 170 is formed in a manner described above, the degree of bending of the sensor assembly 105 may be limited to below the maximum bending strength of the first casing 170. Therefore, it is possible to prevent the sensor assembly 105 from being excessively bent, thereby preventing the sensor panel 110 which is a key element of the sensor assembly 105 from being broken. In addition, since the X-ray detector 100 is bent only to the extent that is under a predetermined limit, the distortion of an obtained X-ray image is reduced.

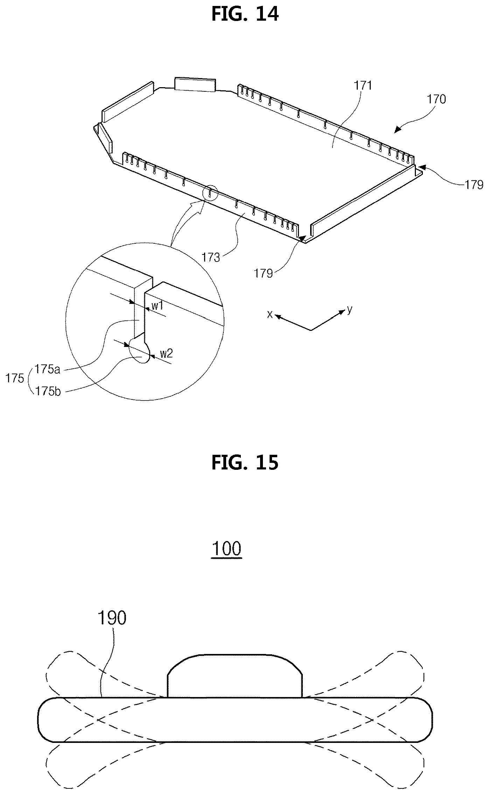

The structure of the first casing 170 will be described in more detail with reference to FIG. 14. The first casing 170 includes a base portion 171 and side walls 173 extending rearward perpendicularly from the respective edges of the back surface of the base portion 171.

The base portion 171 may have a substantially flat plate shape. Preferably, the side walls 173 are not provided at corners of the base portion 171. That is, the side walls 173 adjacent to each other are not connected to each other but are spaced from each other with a gap 179 therebetween, at the corresponding corner of the base portion.

As described above, the side walls 173 are not continuous along the outer periphery of the base portion but are separated from each other with the gaps 179 at the respective corners. Therefore, it is possible to reduce the structural resistance induced at the corners of the first casing 170 and to prevent the first casing 170 from being broken by stress which is usually likely to concentrate on the corners.

The side walls 173 are provided with a plurality of slit-like recesses arranged in a lengthwise direction thereof.

Specifically, among the four side walls 173, two opposite side walls 173 extending along the X-axis (longer-axis) direction of the first casing 170 and facing each other are provided with the recesses 175. The recess formed in one side wall (hereinafter, referred to as a first side wall to differentiate it from the other side wall among the two recesses-provided sidewalls) and the recess formed in the other side wall (hereinafter, referred to as a second side wall) are formed to correspond to each other. The recesses 175 are arranged such that the interval between adjacent recesses is relatively large at a middle portion in the longitudinal direction of the side wall and decreases with a distance to each end.

With the recesses 175 arranged in a manner described above, the degree of bending changes according to positions in the X-axis direction. The smaller the interval of the recesses 175 at a position, the larger the degree of bending at the position. On the contrary, the larger the interval of the recesses 175 at a position, the smaller the degree of bending of the X-ray detector.

That is, the degree of bending increases as the distance to each end of the first casing 170 in the X-axis direction decreases. Therefore, the X-ray detector 100 has a bending characteristic in which the degree of bending varies according to positions in the X-axis direction.

By varying the bending degree according to positions, it is possible to effectively reduce patient discomfort during intraoral radiography.

Actually, respective ends of the X-ray detector 100 frequently come into contact with the intraoral tissues rather than the center portion, so that the respective ends of the X-ray detector 100 mainly cause the discomfort. To solve this problem, the X-ray detector 100 is configured such that its respective end portions are more flexible, which may reduce the discomfort for patients. On the other hand, since a center portion of the X-ray detector 10 is relatively less flexible, the overall distortion of an X-ray image attributable to the bending of the X-ray detector is reduced.

Although the example in which the recesses 175 are formed in the side walls 173 extending in the longer-axis direction has been described above, the recesses 175 may be provided in the side walls 173 extending in the shorter-axis direction as well as the side walls 173 extending in the longer-axis direction. The recesses 175 provided in the side walls 173 extending shorter-axis direction also may vary in interval therebetween.

The recesses 175 provided in the side walls 173 are formed to extend down from the top of each side wall 173. The recesses 175 extending down from the top of the side wall may be stepped recesses, each including a first recess portion 175a having a first width w1 that is a constant width and a second recess portion 125b extending down from a lower end of the first recess portion 175a. At least part of the second recess portion has a second width w2 larger than the first width w1.

The second recess portion 175b may be formed in various shapes. The first embodiment provides an example in which the second recess portion 175b is circular.

The structure in which the second recess portion 175b has a larger width than the first recess portion has the following advantages: preventing a lower portion of the recess 175 provided in the side wall 173 from being damaged during bending of the X-ray detector 100; and improving the bending characteristic of the X-ray detector 100 by allowing the recesses 175 to be expanded more broadly when the X-ray detector 100 is bent.

Alternatively, the recesses 175 starting from the top of the side wall 173 may extend to reach the base portion 171. This case also provides the same advantages. Additionally, the base portion 171 may be provided with a plurality of recesses extending perpendicularly to the lengthwise direction of the X-ray detector 100 in either an outside surface or an inside surface of the base portion 171, as necessary. The surface provided with the recesses may be a surface that comes into the sensor panel 110 or may be a surface that does not come into contact with the sensor panel 110. The intervals of the recesses formed in the surface of the base portion 171 are set in the same manner as the recesses formed in the side walls. That is, the interval decreases with a distance to each end of the base portion. In this case, the recesses formed in the base portion 171 may have a groove form or a slot form that does not pass through the base portion 171 in the thickness direction. When the recesses are formed in the inside surface of the base portion 171, the recesses have a depth less than the thickness of the base portion 171 and are formed to taper to the bottom of the recesses.

The back-surface support 180 is disposed at a rear portion of the sensor assembly 105 and functions as a grip post for supporting the X-ray detector 100 during intraoral radiography. The back-surface support 180 is held by operator's fingers or is connected to an instrument such as an extension cone paralleling (XCP) that is a holder for radiography. The back-surface support 180 may be made of a high-strength resin such as polycarbonate (PC) and acrylonitrile Butadiene styrene (ABS).

As such, the back-surface support 180 is positioned to protect an electrical contact between the signal transmission cable 210 and the printed circuit board 130, so that the electrical contact between the signal transmission cable 210 and the printed circuit board 130 can be stably and reliably maintained.

The back-surface support 180 may be disposed at a center portion of the sensor assembly 105, thereby supporting the rear side of the sensor assembly 105. Therefore, the center portion of the sensor assembly 105 is reduced in the degree of bending compared to the other portions, for example, respective end portions of the sensor assembly 105.

Therefore, the center portion of the X-ray detector 100 is less flexible and the periphery portions of the X-ray detector 100 are more flexible. This characteristic provides effects of reducing patient discomfort and minimizing the distortion of an X-ray radiograph. As such, the back-surface support 180 limits the bending degree of the X-ray detector 100 at the position at which it is disposed, and controls the degree of bending of the X-ray detector 100 according to its arrangement position.

The second casing 200 is structured such that the sensor assembly 105 and the first casing 170 can be received therein. The second casing 200 covers the front side and external flank surfaces of the first casing 170. Besides, the second casing 200 may be structured to cover at least part of the back surface of the sensor assembly 105, as necessary.

The second casing 200 is preferably made of a resin, but the material of the second casing 200 is not limited thereto. Specifically, taking into consideration a bending characteristic that the limit is imposed on the degree of bending of the X-ray detector, the second casing 200 is preferably made of a material having a shore hardness of about D10 to D20, but the material of the second casing 200 is not limited thereto.

When the second casing 200 is made of a material that has the ability to bend within a limited range, it is possible to limit of the overall bending strength of the X-ray detector 100 as intended.

The bending sensor 350 may be disposed to extend along one direction in the X-ray detector 100. For example, the bending sensor 350 may be disposed such that its lengthwise direction is parallel to the longer-axis direction of the X-ray detector 100. When the bending sensor 350 is disposed to extend in the longer-axis direction of the X-ray detector 100, it is possible to measure the degree of bending for the longer-axis direction.

The bending sensor 350 may be disposed at the front side or the rear side of the sensor assembly 105. According to the present embodiment, the bending sensor 350 is disposed at the rear side of the sensor assembly 105. When the bending sensor 350 is disposed at the rear side of the sensor assembly 105, the bending sensor 350 can avoid interference of incidence X-rays.

Alternatively, the bending sensor 350 may be disposed inside the sensor assembly 105.

Alternatively, two bending sensors 350 arranged to be perpendicular to each other may be used. A first bending sensor and a second bending sensor constituting the two bending sensors 350 may be disposed to extend along the longer-axis direction and the shorter-axis direction, respectively. In this case, it is possible to detect the degree of bending in both of the longer-axis direction and the shorter-axis direction of the X-ray detector 100.

When the signal generator circuit 400 is disposed outside the X-ray detector 100, the bending sensor 350 may be electrically connected to the signal generator circuit 400 disposed outside the X-ray detector 100 via the signal transmission cable 210.

The X-ray detector 100 with the second casing 200 combined therewith may subsequently undergo a molding process so that at least part of the X-ray detector 100 may be covered by a soft housing member 190. The housing member 190 encloses the external surface of the X-ray detector 100, thereby protecting the X-ray detector 100.

The housing member 190 may be formed to cover the entire surface of the sensor assembly 105. Additionally, the housing member 190 also may cover the second casing 200 and/or the back-surface support 180, as necessary.

The housing member 190 may be made of a soft material such as silicone or urethane. Preferably, the soft material for the housing member 190 may have a shore hardness of about A30 to A50. However, the soft material for the housing member 190 is not limited thereto.

FIG. 15 is a diagram illustrating a state in which bending of the X-ray detector according to one embodiment of the present invention occurs. Referring to FIG. 15, when the X-ray detector 100 is bent, it is possible to detect the degree of bending by using the bending sensor (refer to reference numeral 350 in FIG. 11). Therefore, when excessive bending which may cause damage to the X-ray detector 100 occurs, a warning voice sound or a warning display message may be output to prevent the X-ray detector 100 from being damaged.

## Claims

1. An X-ray detector having a flexible property and comprising a pressure sensor measuring a magnitude of an external force applied thereto.

2. The X-ray detector according to claim 1, wherein the pressure sensor changes in color according to the magnitude of the external force.

3. The X-ray detector according to claim 1, further comprising a sensor panel configured to convert an X-ray incident onto a front surface thereto into an electrical signal, wherein the pressure sensor is disposed at a front side or a rear side of the sensor panel.

4. The X-ray detector according to claim 3, wherein the pressure sensor is directly attached to the sensor panel.

5. The X-ray detector according to claim 2, further comprising:
a first casing configured to cover a front portion of the sensor panel and to limit an elastic bending of the sensor panel;
a second casing provided at a front side of the first casing and accommodating the sensor panel and the first casing; and
a bending control member disposed at a rear side of the sensor panel and controlling the elastic bending of the sensor panel.

6. The X-ray detector according to any one of claims 1 to 5, further comprising a soft housing member covering an outside surface of the X-ray detector.

7. A bending detection system for detecting a degree of bending of an X-ray detector, the system comprising:
an X-ray detector being flexible and comprising a sensor panel;
a bending sensor provided in the X-ray detector and changing in resistance according to a degree of bending of the X-ray detector;
a signal generator circuit generating a bending signal corresponding to a change in the resistance of the bending sensor;
a bending information generator circuit generating a defection information signal according to the bending signal and transmitting the resulting bending information signal to an output device.

8. The system according to claim 7, wherein the signal generator circuit comprises: a resistor connected in series with the bending sensor to configure a voltage divider circuit in conjunction with the bending sensor, and an analog-digital converter circuit converting an output voltage of the voltage divider circuit to a digital signal serving as the bending signal.

9. The system according to claim 8, wherein the signal generator circuit further comprises an operational amplifier amplifying the output voltage of the voltage divider circuit and outputting the resulting amplified signal to the analog-digital converter circuit.

10. The system according to claim 7, wherein the bending information generator circuit generates a voice information signal indicating the degree of bending and transmits the resulting voice information signal to a voice output device, or the bending information generator circuit generates a display information signal indicating the degree of bending and transmits the display information signal to a display device.

11. The system according to claim 7, wherein the X-ray detector further comprises a first casing covering a front portion of the sensor panel and limiting an elastic bending of the sensor panel.

12. The system according to claim 7, wherein the X-ray detector further comprises a bending control member disposed at a rear side of the sensor panel and controlling the degree of bending of the sensor panel.
